# NEUE EUROPÄISCHE PATENTSCHRIFT

(11) **EP 2 482 866 B2**
(45) Veröffentlichungstag und Bekanntmachung des Hinweises auf die Entscheidung über den Einspruch: **11.09.2024**
(45) Hinweis auf die Patenterteilung: 22.07.2015
(21) Anmeldenummer: 10759895.5
(22) Anmeldetag: 29.09.2010
(51) Int. Cl.: A61M 1/14

(54) **GEHÄUSE MIT VESCHLUSSKLAPPE**
HOUSING WITH CLOSURE FLAP
BOÎTIER POURVU D'UN CLAPET DE FERMETURE

(30) Priorität: 29.09.2009 DE 102009045095
(43) Veröffentlichungstag der Anmeldung: 08.08.2012
(73) Patentinhaber: Fresenius Medical Care Deutschland GmbH, 61352 Bad Homburg (DE)
(72) Erfinder: SYFONIOS, Andreas, 97493 Bergrheinfeld (DE)
(74) Vertreter: Nordmeyer, Philipp Werner
(86) Internationale Anmeldenummer: PCT/EP2010/064448
(87) Internationale Veröffentlichungsnummer: WO 2011/039248

(56) Entgegenhaltungen:
- US-A- 5 482 440
- US-A1- 2002 147 423
- US-A1- 2008 018 131
- US-A1- 2009 107 335
- US-A1- 2009 107 335
- "Fachpflege Nephrologie und Dialyse", München, ISBN: 978-3-437-26252-4, article G. BREUCH, pages: 138,271
- SERVICE-HANDBUCH DER PATENTINHABERIN ZUM DIALYSEGERÄT DES TYPS 5008
- G . BREUCH ET AL., DIALYSE FÜR EINSTEIGER, 2007, Mün- chen, ISBN: 978-3-437-27790-0

## Beschreibung

Die Erfindung bezieht sich auf ein Gerätegehäuse für medizinische Geräte mit einer Verschlussklappe für Servicezwecke.

Derartige Gerätegehäuse werden häufig bei Blutbehandlungsgeräten wie bspw. Hämodialysemaschinen eingesetzt. Hämodialyse umfasst auch Hämofiltration und Hämodifiltration.

Bei diesen Geräten ist es notwendig, im Servicefall einfach an das Innere des Geräts zu gelangen. Dazu wird eine Verschlussklappe, welche über ein Scharnier oder Gelenk an einer Seite oder der Front des Geräts befestigt ist, aus dem Gehäuse ausgeklappt. An dieser Verschlussklappe sind üblicherweise technische Einrichtungen, wie z.B. das extrakorporale Blutbehandlungsmodul (EBM) befestigt. So kann ein Servicetechniker einfach an das Geräteinnere und an alle Seiten des EBM gelangen, da letzteres aufgrund des Aufklappens zumindest großteils außerhalb des Geräteinneren befindlich ist.

Im Hausgerätebau sind, wie unter DE 10 2005 021 540 A1 oder EP 1 535 556 B1 gezeigt, auch Scharniere bekannt, welche eine Schwenk- und Schiebebewegung verbinden. Auch lassen sich gemäß WO 2008/010004 A1 über mehrgliedrige Koppelgetriebe komplexe Bewegungen von Klappen oder Deckeln realisieren.

Bei bekannten Bauformen der Verschlussklappe von Blutbehandlungsgeräten wird üblicherweise lediglich eine Schwenkachse vorgesehen. Dabei ist die Front der Verschlussklappe, an der sich diverse Einstell- und Anzeigeeinheiten befinden, im ausgeklappten Zustand nach unten geneigt, so dass sie von dem Servicetechniker schlecht einsehbar ist.

Es ist die Aufgabe der Erfindung die bekannten Nachteile zu vermeiden und ein kostengünstiges und robustes Gehäuse bereitzustellen, bei welcher der Techniker die benötigten Serviceaufgaben im Geräteinneren und den an der Verschlussklappe befindlichen Komponenten vornehmen und gleichzeitig die an der Vorderseite der Verschlussklappe befindlichen Bedienkomponenten gut erreichen kann.

US 2009/0107335 A1 betrifft eine Luftfalle für eine medizinische Infusionsvorrichtung.

US 5,482,440 betrifft ein Blutbehandlungssystem unter Verwendung eines peristaltischen Pumpmoduls mit einem Ventil.

US 2008/0018131 A1 betrifft ein Schwanenhalsscharnier für ein Kraftfahrzeug.

US 2002/0147423 A1 betrifft Systeme und Verfahren zum Durchführen einer Blutbehandlung und/oder zum Durchführen von Fluidaustauschverfahren.

Diese Aufgabe wird durch das Gerätegehäuse mit den Merkmalen des Anspruchs 1 gelöst. Weiterbildende Ausführungsformen sind in den Unteransprüchen angegeben.

Das erfindungsgemäße Gerätegehäuse eines Blutbehandlungsgeräts weist eine Gehäusewand und eine Verschlussklappe zum Verschließen einer Öffnung der Gehäusewand in einer ersten Position auf. Dabei ist ein Koppelelement über eine erste Drehachse gegen die Gehäusewand drehbar gelagert und die Verschlussklappe ist über eine zweite Drehachse gegen das Koppelelement drehbar gelagert. Dabei ist die Verschlussklappe zumindest in einer definierten zweiten Arbeitsposition einrichtbar, bei der die Öffnung der Gehäusewand nicht verschlossen ist. Die Verschlussklappe weist zumindest Teile eines extrakorporalen Blutbehandlungsmoduls umfassend mindestens eine Pumpe und/oder einen Durchflussmesser und/oder ein Ventil und/oder eine Absperrklemme auf und ist derart eingerichtet, dass diese der Schwerkraft folgend in die zweite Arbeitsposition wegklappbar, ist. Vorteilhafterweise ist die Verschlussklappe auch in einer dritten definierten Arbeitsposition einrichtbar, bei der die Öffnung der Gehäusewand nicht verschlossen ist. Die Öffnung ist bspw. eine Serviceöffnung, durch die eine Serviceperson das Innere des Geräts oder an der Rückseite der Verschlussklappe befindliche Komponenten erreichen kann. Die erste und zweite Drehachse sind vorzugsweise parallel und komplanar oder parallel zu der Gehäuseöffnung und/oder der Verschlussklappe ausgerichtet. Ferner ist die erste Drehachse insbesondere nicht kollinear zu der zweiten Drehachse; sie haben also einen seitlichen Versatz. Die Arbeitspositionen werden als je eine definierte Lage der Verschlussklappe verstanden, welche entweder über Haltemittel, wie Rasten, Magnete, Hebel, Riegel, Fanggurte o.ä. oder der Schwerkraft bestimmt werden und bei der die Verschlussklappe aufgrund der genannten Mittel in dieser Lage gehalten wird. Während bei einer bekannten Klappe nur eine Drehachse vorgesehen ist, so kann durch die zusätzliche zweite Drehachse die Winkellage der Verschlussklappe in der Lage, bei der das Koppelelement in einer komplett ausgeklappten Lage ist, verändert und so eingerichtet werden, dass die Serviceperson neben den Servicebereichen auch die Front mit den Bedien- und Steuerelementen gut erreichen. Da diese Arbeitspositionen eine Lagestabilität aufweisen, kann die Serviceperson gut in diesen Lagen an der Verschlussklappe arbeiten, Einstellungen vornehmen und das Geräteinnere erreichen.

Vorteilhafterweise ist die Öffnung an einer Gehäusewand angebracht, welche im Wesentlichen vertikal ausgerichtet ist. So kann die Verschlussklappe nach unten weggeklappt werden und der Schwerkraft folgend in eine der entsprechenden Arbeitspositionen gebracht werden. Da die Verschlussklappe in der geschlossenen Position die Öffnung verschließt, sind die an der Verschlussklappe angeformten Bedien- und Steuerelemente für den Benutzer in dieser Lage ebenfalls vertikal ausgerichtet und somit gut ablesbar.

Weiterführend kann die Verschlussklappe über keine weiteren Drehachsen und/oder Koppelelemente gegen das Gerätegehäuse gelagert sein. Diese Formulierung umfasst, dass sowohl an der Verschlussklappe als auch dem Koppelelement keine weiteren zwischengeschalteten Drehachsen vorgesehen sind. Das erfindungsgemäße Koppelelement kann aber mehrteilig sein, wie etwa durch mehrere parallel angeordnete baugleiche oder -ähnliche Teile mit Lagerpunkten die auf den gleichen Drehachsen liegen. Während es in der Mechanik bekannt ist, Komponenten über mehrgliedrige Koppelelemente, wie z.B. viergliedrige Koppelgetriebe, zu führen, wird bei der vorgeschlagenen Konstruktion eine einfache und kostengünstige Lösung bereitgestellt.

Ferner wird weiterführend auch die Verschlussklappe bei ihrer Bewegung über keine Linearführungen gegen das Gerätegehäuse geführt. Eine Linearführung beinhaltet i.d.R. die Gefahr des Verkantens der Führung, ist mit erhöhten Herstellkosten verbunden und eher gegen Verschleiß anfällig. So ergibt sich vorliegend ein Vorteil der exakten und kostengünstigen Führung. Diese Formulierung umfasst, dass vorzugsweise sowohl an der Verschlussklappe, wie auch dem Koppelelement keine Lagerungen in Form einer Linearführung vorgesehen sind.

In einer weiteren Ausgestaltung ist ein Rastmechanismus zwischen dem Koppelelement und der Verschlussklappe vorgesehen ist. Vorzugsweise ist, wenn der Rastmechanismus in einer rastenden Stellung ist, die Verschlussklappe in der zweiten Arbeitsposition im Wesentlichen vertikal ausgerichtet. Im Wesentlichen vertikal umfasst insbesondere Winkelabweichungen von +/- 15°, vorzugsweise +/-5° gegen die Vertikale. Durch die vertikale Ausrichtung kann die Serviceperson die Bedien- und Steuerelemente gut ablesen und die Verschlussklappe ist gegen ein Herausschwenken aus dieser Stellung gesichert.

Der Rastmechanismus kann insbesondere durch manuell verschließ- und/oder öffenbare Riegel oder Verhakungen oder durch federgestützte Rasten oder magnetische oder reibhaftende Mittel oder Ausnutzung der Schwerkraft realisiert sein. Der Rastmechanismus kann auch mehrere rastende Stellungen aufweisen.

Im Fall, dass die Gehäusewand des Gehäuses leicht schräg, nach oben geneigt zulaufend ausgeführt und/oder entsprechend gewölbt ist, ist es vorteilhaft, die Neigung der Verschlussklappe parallel zu der Gehäusewand auszuführen.

Vorteilhafterweise tendiert die Verschlussklappe aufgrund der Schwerkraft dazu, von der zweiten Arbeitsposition in die dritte Arbeitsposition zu schwenken. Dabei ist die dritte stabile Arbeitsposition eine Lage, bei der die Öffnung der Gehäusewand geöffnet ist und die Verschlussklappe nicht in der vertikalen Lage ist. An der dritten Arbeitsstation kann alternativ ein oben beschriebener Rastmechanismus vorgesehen sein.

Vorteilhafterweise kann die Lagerung der Verschlussklappe gegen das Koppelelement einen Längsschlitz aufweisen, über den die Verschlussklappe gegenüber dem Koppelelement lösbar und insbesondere aushebbar ist. Ein derartiger Längsschlitz kann als U-förmige Aufnahme so in das Koppelelement geformt sein, dass eine Achse oder Bolzen der Verschlussklappe aufgrund der Schwerkraft in den Grund des "U"s drängt. Für Service- und Austauschzwecke ist so die Verschlussklappe durch Anheben leicht entfernbar. Selbstverständlich ist eine Funktionsumkehr durch einen wechselseitigen Austausch der Aufnahme und des Bolzens möglich. Ggf. ist dieses Entfernen erst nach Öffnen eines Verschlusselements, wie eines Riegels o.ä. möglich. Auch kann ein Anschlag vorgesehen sein, der ein einfaches Herausrutschen aus der U-förmigen Aufnahme verhindert und die Entnahme nur über eine mehrstufige Bewegung in unterschiedlichen Richtungen erlaubt.

Zur Verbesserung der Festigkeit und insbesondere Verwindungssteifigkeit können auch zwei oder mehr im Wesentlichen spiegelsymmetrische oder identische Koppelelemente vorgesehen sein, die parallel zueinander ausgerichtet sind. Hierbei sind die Achsen der Lagerungen jeweils kollinear.

In einer weiterführenden Ausgestaltung kann das Koppelelement bogenförmig sein und ein unterer Schenkel des Bogens verbleibt in allen drei Positionen innerhalb des Gerätegehäuses und ein oberer Schenkel des Bogens wird in den geöffneten Arbeitspositionen durch die Öffnung aus dem Gehäuse geführt. Der Begriff "Bogen" ist hier weit zu verstehen und soll insbesondere jede mechanische Ausgestaltung des Koppelelements umfassen, bei der die Lagerstellen der Drehachsen so miteinander verbunden sind, dass in ihrer Mitte zumindest teilweise eine lichte Weite entsteht, in welche beim Übergang in eine der geöffneten Arbeitspositionen die Gehäusewand teilweise aufgenommen werden kann. Durch diese Ausgestaltung kann das Koppelelement unterhalb der genannten Öffnung komplett innerhalb des Gehäuses geführt werden und so ergibt sich ein optischer, designmäßiger Vorteil.

Vorteilhafterweise sind an der Außenseite der Verschlussklappe Bedienelemente des Geräts angeordnet. Hierzu zählen elektrische oder mechanische Einstelleinrichtungen, Bedienelemente, Anzeigen oder (Schlauch-)Pumpen, Verschlüsse, Ventile oder ähnliches.

Ferner kann der Zugangsbereich zu der Öffnung der Gehäusewand in der dritten Arbeitsposition größer sein als in der zweiten Arbeitsposition. Der genannte Zugangsbereich wird als der Raum vor der Öffnung im Abstand von 0 bis 60 cm von der Gehäusewand nach außen definiert. Durch das Wegklappen der Verschlussklappe von der zweiten in die dritte Arbeitsposition um die zweite Drehachse wird dieser Raum größer und somit das Innere des Gehäuses besser für Montageaufgaben erreichbar. Wenn lediglich eine einzige (untere) Drehachse vorhanden wäre, so ließe sich der Zugangsbereich nur dadurch vergrößern, dass der Kippwinkel dieser Drehachse vergrößert würde. Dies bringt aber den Nachteil, dass die Entfernung von der Verschlussklappe zum Gehäuse auch größer würde, was das Problem erzeugt, dass die Anschlusskabel und -schläuche entsprechend größer ausgelegt werden müssten. Durch die definierte dritte Arbeitsposition wird zum einen die Öffnung besser frei gegeben und dies ohne dass der genannte Abstand, also die benötigte Verbindungslänge der Versorgungsleitungen, entsprechend erhöht werden muss.

Auch kann in der dritten Arbeitsposition der Öffnungswinkel der Verschlussklappe größer sein als der Öffnungswinkel des Koppelelements. Der Öffnungswinkel wird verstanden als die Winkeldifferenz der Drehung des jeweiligen Elements von der ersten Position in die dritte Arbeitsposition. Dieser Winkel ist nicht die Relativbewegung in der Drehachse, sondern wird auf eine ortsfeste Bezugsgröße, wie z.B. die Seitenwand bezogen. Aufgrund dieser Winkellage ist die Rückseite der an der Innenseite der Verschlussklappe befindlichen Anbauteile besser in der ausgeklappten dritten Arbeitsposition von oben für Servicezwecke zugänglich.

Anhand der Zeichnung wird die Erfindung erläutert. Es zeigen:
- Fig. 1: eine Prinzipskizze einer Seitenansicht des Gerätegehäuses in der dritten Arbeitsposition, bei der die Verschlussklappe geöffnet ist und die Verschlussklappe nicht vertikal ausgerichtet ist,
- Fig. 2: die Prinzipskizze in einer zweiten Arbeitsposition, bei der ein Rastmechanismus die Verschlussklappe vertikal oder senkrecht hält,
- Fig. 3: die Prinzipskizze in einer ersten Position, bei der die Verschlussklappe geschlossen ist,
- Fig. 4: eine alternative Ausführungsform der zweiten Arbeitsposition,
- Fig. 5: eine alternative Ausführungsform der dritten Arbeitsposition,
- Fig. 6: eine konkrete Ausgestaltung des Koppelelements,
- Fig. 7: eine Variante der Fig. 2 mit schräger Front der Seitenwand und.
- Fig. 8: die Lagerung der Koppelelemente 30.

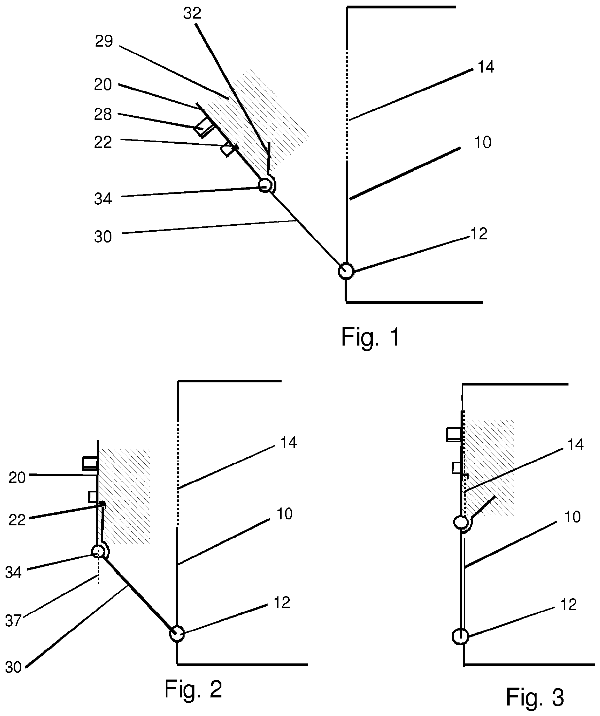

In der Prinzipskizze gemäß Fig. 1 wird die Erfindung vereinfacht dargestellt. Sie zeigt eine Seitenansicht einer Gehäusewand 10, welche üblicherweise als die Gerätefront ausgeführt ist, an welcher sich dem Benutzer zugängliche Bedienteile 28 befinden. In der Gehäusewand 10 ist eine Öffnung 14 vorgesehen, welche gestrichelt angedeutet ist, und über die der Servicetechniker das Innere des Gerätes erreichen kann. Diese Öffnung 14 kann mit der Verschlussklappe 20 verschlossen werden.

An der Verschlussklappe 20 sind zumindest Teiles eines extrakorporalen Blutbehandlungsmoduls EBM vorgesehen. Dabei sind an der Vorderseite der Bedienklappe des Blutbehandlungsgeräts Bedien- und Steuerelemente 28, wie Pumpen, insbesondere Blut- und/oder Heparinpumpen, Durchflussmesser, Anzeigen, Ventile, Absperrklemmen, Tropfkammern, Schlauchhalter, Sensoren (Druck-, Leitfähigkeits-, Temperatur-, Pegel-, optische Sensoren etc.) und Filter, angeordnet. An der Rückseite der Bedienklappe sind die entsprechenden elektrischen Antriebe der Pumpen, Ver- und Entsorgungsschlauchsysteme und elektronische Steuer- und Kontrolleinheiten vorgesehen.

Die Verschlussklappe 20 kann weiter dazu eingerichtet sein, eine medizinische Behandlungskassette aufzunehmen. Entsprechend können an der Verschlussklappe 20 sowohl Halterungen zur mechanischen Aufnahme als auch Aktoren und Sensoren, bzw. elektrische Schnittstellen vorhanden sein, um mit entsprechenden Mitteln in der medizinischen Behandlungskassette zu interagieren.

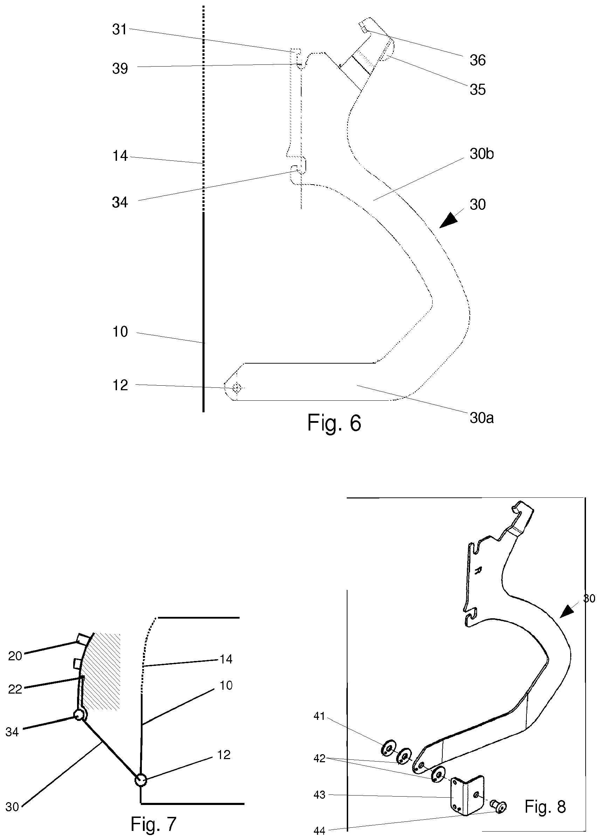

Wenn die Verschlussklappe 20 die Öffnung 14 verschließt, werden die rückseitigen Bestandteile 29 des EBM komplett im Gerät aufgenommen. Dies entspricht der üblichen Arbeitsweise des Geräts und ist in Fig. 3 dargestellt. An einem unteren Bereich der Verschlussklappe 20 ist ein Drehpunkt einer zweiten Drehachse 34 vorgesehen, an dem ein Koppelelement 30 angreift. Dieses Koppelelement 30 ist als eine Koppelstange oder ein entsprechendes Stanzteil oder lasergeschnittenes Teil ausgeführt (siehe Fig. 6). Dieses Koppelelement 30 umfasst einen unteren Lagerpunkt, welcher als erste Drehachse 12 gegen das Gehäuse gelagert ist. Die Verschlussklappe 20 ist also über die zwei Freiheitsgrade der zwei Drehachsen 34 und 12 gegen das Gehäuse schwenkbar. In einer praktischen Ausführungsform wird vorzugsweise das Koppelelement 30 in Form von zwei parallelen und im Wesentlichen symmetrischen Stanzteilen ausgeführt, um so die Führung und Verwindungssteifigkeit zu erhöhen.

Von der in Fig. 3 gezeigten ersten geschlossenen Position lässt sich die Verschlussklappe 20 nach dem Öffnen von nicht dargestellten Verschlusselementen, wie Riegeln oder Rasten um die erste Drehachse 12 aufklappen und gelangt so in die dritte Arbeitsposition gemäß Fig. 1. Hier ist die Front der Verschlussklappe 20 mit den Bedien- und Steuerelementen 28 nach unten geneigt und das EBM liegt außerhalb des Gehäuses und ist von oben und den Seiten gut für Serviceaufgaben zugänglich. Ein nicht dargestellter Anschlag zwischen der Verschlussklappe 20 und dem Koppelelement 30 sorgt dafür, dass die Verschlussklappe 20 nicht aufgrund der Schwerkraft weiter nach unten schwenkt. Ebenso sorgt ein nicht dargestellter Anschlag zwischen dem Koppelelement 30 und dem Gehäuse dafür, dass seine Schwenkbewegung begrenzt wird. Ein Halteband oder Fanggurt 17 kann bevorzugt zwischen dem Gehäuse und der Verschlussklappe 20 befestigt sein und in der ausgeklappten Stellung (siehe z.B. Fig. 1) zumindest einen Teil der Gewichtskräfte aufnehmen.

Das bislang beschriebene Schwenken ist ein Schwenken ausschließlich um die erste Drehachse 12. Wenn nun die Verschlussklappe 20 entgegen dieser Schwenkrichtung um die zweite Schwenkachse 34 geschwenkt wird, so gelangt die Verschlussklappe 20 in die zweite geöffnete Arbeitsposition gemäß Fig. 2, bei der die Verschlussklappe 20 vertikal ausgerichtet ist, das EBM weiterhin gut erreichbar ist und die Bedien- und Steuerelemente 28 vertikal nach vorne ausgerichtet. Anders als in der dritten Arbeitsposition sind die Bedien- und Steuerelemente 28 nicht nach unten geneigt. In dieser zweiten Arbeitsposition kann der Servicetechniker die üblichen Bedienvorgänge des Benutzers gut nachvollziehen und gleichzeitig die notwendigen Serviceaufgaben vornehmen.

Die erste, zweite und dritte Position sind jeweils stabile Lagen. Das heißt, es muss ein Widerstand in Form einer Rast überwunden werden, oder ein Riegel geöffnet oder eine Bewegung entgegen der Schwerkraft durchgeführt werden, um die Verschlussklappe aus dieser stabilen Lage zu bringen.

Es sei betont, dass die Fig. 1 - 5 Prinzipskizzen sind. So muss bspw. die zweite Drehachse 34 nicht unterhalb der Verschlussklappe 20 angeordnet sein, sondern die Verschlussklappe kann sich auch unterhalb der zweiten Drehachse 34 erstrecken, wie dies durch die Verlängerung 37 der Verschlussklappe 20 nach unten in Fig. 2 dargestellt ist. Ebenfalls können die Drehachsen 12, 34 nach innen versetzt sein, so dass die entsprechenden Lagerungen in der geschlossenen Position gemäß Fig. 3 komplett im Gehäuse aufgenommen und von außen nicht sichtbar sind. Dies ist bspw. bei dem Lagerpunkt der ersten Drehachse 12 gemäß Fig. 6 gezeigt.

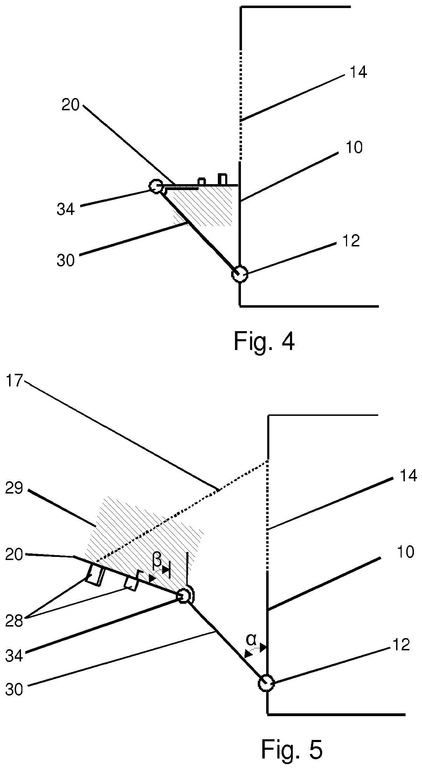

Aus Fig. 5 ist ein Vorteil der Erfindung gegenüber dem Stand der Technik ersichtlich. Bekannt aus dem Stand der Technik waren Ausführungen mit einer ersten Drehachse 12 und ohne eine zweite Drehachse 34. Gemäß Fig. 5 kann durch die zweite Drehachse 34 der Öffnungswinkel β größer als der Öffnungswinkel α sein, so dass die Serviceperson gut von oben die Rückseite des EBM erreichen kann. Dies war bislang nicht möglich, da aufgrund der Begrenzungen der Kabel- und Schlauchlängen das EBM nicht über einen größeren unteren Öffnungswinkel α weiter vorgeklappt werden konnte. Durch die beiden Drehachsen kann ein größerer Öffnungswinkel β erreicht werden, ohne dass das EBM weit von dem Gehäuse entfernt ist.

Die Ausführung gemäß Fig. 2, bei der die Verschlussklappe 20 senkrecht ausgerichtet ist, ist nur ein Beispiel. Ebenso können andere Serviceausrichtungen bspw. senkrecht +/- 10° oder +/- 30° vorteilhaft sein oder die alternative Ausführung der Fig. 4, bei der die Verschlussklappe 20 mit ihrer Frontseite nach oben zeigt. So kann eine Serviceperson im letzteren Fall gut zum einen durch die Öffnung 14 das Geräteinnere erreichen und gleichzeitig die Bedien- und Steuerelemente 28 der Verschlussklappe 20 erreichen, ablesen und bedienen.

Fig. 7 zeigt eine Variante, bei der die Seitenwand 10 im Bereich der Öffnung 14 nach oben geneigt ist. Zusätzlich ist eine Rundung als eine konvexe Kontur vorgesehen. Dadurch wird dem Gerät ein ansprechendes Design verliehen und da die Verschlussklappe 20 etwa in Brusthöhe einer vor ihr stehenden Person liegt, sind an der Verschlussklappe 20 befindliche Bedienteile besser zu erreichen. Nach dem Öffnen ist die Winkellage der Verschlussklappe 20, wie in Fig. 7 dargestellt, identisch zu der Ausrichtung des geschlossenen Zustands. Es wurde also ein Parallelversatz durchgeführt.

Ferner ist in Fig. 5 ein Fanggurt 17 dargestellt, welcher zum einen an der Gehäusewand 10 oder im Gehäuse gehalten und zum anderen an der Verschlussklappe 20 befestigt ist. Dieser Fanggurt 17 ist als ein biegeschlaffes Element, wie Seil, Band oder Gurt ausgeführt und steht gemäß Fig. 5 unter Zugspannung, nimmt so Gewichtskräfte der Verschlussklappe 20 und des EBM 29 auf und sorgt für die Begrenzung des Öffnungswinkels β. Auch kann durch den Fanggurt 17 der Öffnungswinkel α begrenzt werden.

Fig. 6 zeigt eine konkrete Ausgestaltung des Koppelelements 30, von dem vorzugsweise zwei Stück baugleich oder symmetrisch ausgeführt und parallel ausgerichtet sind und zusammenwirkend die Verschlussklappe 20 lagern. Gemäß den Prinzipien der Fig. 1 - 5 erscheint es, als ob das Koppelelement 30 auch in der geschlossenen ersten Position von außen sichtbar sei. Dies ist aber nicht zwingend nötig, da das Koppelelement 30 auch gemäß Fig. 6 bogenförmig ausgeführt sein kann. Hierbei ist ein unterer Schenkel 30a stets im Gehäuse, während ein oberer Schenkel 30b für die geöffneten Arbeitspositionen durch die Öffnung 14 aus dem Gehäuse herausschwenken kann. So wird der optische Eindruck der Außenansicht des Geräts unterhalb der Verschlussklappe 20 nicht durch ein sichtbares Koppelelement 30 gestört. Fig. 6 zeigt ferner einen Anschlag 31, welcher den Schlitz 39 nach oben begrenzt. Wenn der Schlitz 39 zusätzlich zur Lagerung um die Drehachse 34 verwendet wird, so sorgt der Anschlag 31 dafür, dass bei einer Entnahme des EBM eine doppelte Bewegung, also ein Anheben und ein seitliches Versetzen durchgeführt werden muss, um so ein unbeabsichtigtes Herausfallen, wie bei einem Anstoßen von unten zu verhindern.

Das Koppelelement 30 ist als Stanz- und Biegeteil aus Metall mit einer ausreichenden Dicke, z. B. min. 2 mm +/- 1 mm ausgeführt und weist an einem oberen Schenkel 30b einen Entriegler 35 auf, und eine durch Biegen erzeugte Abwinklung 36, welche mit einem entsprechenden Anschlag (nicht dargestellt) an der Verschlussklappe 20 in Eingriff bringbar ist und so die Verschlussklappe 20 in einer vertikalen Arbeitsposition gemäß Fig. 2 halten kann. Wenn nun die Serviceperson den Entriegler 35 (gemäß Fig. 6 in die Zeichenebene) drückt, so kann sich das Koppelelement 30 leicht elastisch verformen, so dass die Abwinklung 36 wie ein Riegel aus dem genannten Anschlag entfernbar wird und die Verschlussklappe 20 in Folge entlang der zweiten Drehachse 34 schwenkbar ist. Auf diese Weise können kostengünstig, wie z.B. durch ein lasergeschnittenes Werkstück, zum einen die Lagerung der Verschlussklappe 20 und auch die Definition der Arbeitspositionen in einem einstückigen Koppelelement 30 realisiert werden.

Fig. 8 zeigt die Lagerung des Koppelelements 30 über einen Lagerträger 43, der am und vorzugsweise im Gerätegehäuse befestigt ist. Zu beiden Seiten des Koppelelements 30 befinden sich Kunststoffscheiben 42, wobei zu der einen Seite zusätzlich eine Unterlegscheibe 41 vorgesehen ist. Zu der anderen Seite wird eine Niet 44 eingesetzt, welche die genannten Elemente unter axialer Vorspannung hält. Dadurch wird neben der Lagerung eine Bremsung oder eine gewisse Dämpfung des Koppelelements 30 erreicht. Da das EBM mit ca. 15 kg ein nennenswertes Gewicht aufweist, ist diese Bremsung oder Dämpfung sinnvoll, da bei ungedämpfter Mechanik beim Erreichen der Endlage hohe Kräfte auf die Konstruktion ausgeübt würden und auch die Gefahr des Herausfallens des EBMs bestünde. Zusätzlich kann die Dämpfung durch Gasdruckfedern/-dämpfer (nicht dargestellt) unterstützt werden. Weiter erleichtern die Kunststoffscheiben 42 durch den Bewegungswiderstand, welchen sie auf das Koppelelement 30 ausüben, das Einführen des EBMs bei Service- und Montagetätigkeiten in die entsprechenden Aufnahmen. Das Einführen eines Lagerbolzens des EBMs in die entsprechende Aufnahme des Koppelelements 30 wird nämlich dadurch erleichtert, dass das Koppelelement 30 durch die genannte Bremsung lagestabil bleibt.

Merkmale unterschiedlicher Ausführungsformen sind frei miteinander kombinierbar. Insbesondere sind die Lagen und Definitionen der unterschiedlichen Arbeitspositionen frei austauschbar und mehr als die drei genannten stabilen Arbeitspositionen sind vom Erfindungsgedanken umfasst.

## Patentansprüche

1. Gerätegehäuse für ein Blutbehandlungsgerät, mit einer Gehäusewand (10) und einer Verschlussklappe (20), welche eine Öffnung (14) in der Gehäusewand (10) in einer ersten Position verschließt und wobei ein Koppelelement (30) über eine erste Drehachse (12) gegen die Gehäusewand (10) drehbar gelagert ist und die Verschlussklappe (20) über eine zweite Drehachse (34) gegen das Koppelelement (30) drehbar gelagert ist, wobei die Verschlussklappe (20) zumindest in einer definierten zweiten Arbeitsposition einrichtbar ist, bei der die Öffnung (14) der Gehäusewand nicht verschlossen ist,
wobei die Verschlussklappe (20) zumindest Teile eines extrakorporalen Blutbehandlungsmoduls (28, 29) umfassend mindestens eine Pumpe und/oder einen Durchflussmesser und/oder ein Ventil und/oder eine Absperrklemme aufweist,
**dadurch gekennzeichnet, dass**
die Verschlussklappe (20) derart eingerichtet ist, dass diese der Schwerkraft folgend in die zweite Arbeitsposition wegklappbar ist.

2. Gerätegehäuse gemäß Anspruch 1, **dadurch gekennzeichnet, dass** die Verschlussklappe (20) ferner in einer definierten dritten Arbeitsposition einrichtbar ist, bei der die Öffnung (14) der Gehäusewand nicht verschlossen ist.

3. Gerätegehäuse gemäß einem der vorangegangenen Ansprüche, **dadurch gekennzeichnet, dass** die Gehäusewand (10) im Wesentlichen vertikal ausgerichtet ist.

4. Gerätegehäuse gemäß einem der vorangegangenen Ansprüche, **dadurch gekennzeichnet, dass** die Verschlussklappe über keine weiteren Drehachsen und/oder Koppelelemente gegen das Gerätegehäuse gelagert ist.

5. Gerätegehäuse gemäß einem der vorangegangenen Ansprüche, **dadurch gekennzeichnet, dass** die Verschlussklappe (20) über keine Linearführungen gegen das Gerätegehäuse geführt ist.

6. Gerätegehäuse gemäß einem der vorangegangenen Ansprüche, **dadurch gekennzeichnet, dass** ein Rastmechanismus (22, 32; 36) zwischen dem Koppelelement (30) und der Verschlussklappe (20) vorgesehen ist, und insbesondere der Rastmechanismus (22, 32; 36) so eingerichtet ist, dass die Verschlussklappe (20) in der zweiten Arbeitsposition im Wesentlichen vertikal ausgerichtet ist, wenn der Rastmechanismus (22, 32) in einer rastenden Stellung ist.

7. Gerätegehäuse gemäß einem der vorangegangenen Ansprüche 1 - 4, **dadurch gekennzeichnet, dass** ein Rastmechanismus (22, 32; 36) zwischen dem Koppelelement (30) und der Verschlussklappe (20) vorgesehen ist, und insbesondere der Rastmechanismus (22, 32; 36) so eingerichtet ist, dass die Verschlussklappe (20) in der zweiten Arbeitsposition im Wesentlichen parallel zu der Gehäusewand (10) ausgerichtet ist, wenn der Rastmechanismus (22, 32) in einer rastenden Stellung ist.

8. Gerätegehäuse gemäß einem der vorangegangenen Ansprüche, **dadurch gekennzeichnet, dass** die Verschlussklappe (20) aufgrund der Schwerkraft dazu tendiert von der zweiten Arbeitsposition in die dritte Arbeitsposition zu schwenken.

9. Gerätegehäuse gemäß einem der vorangegangenen Ansprüche, **dadurch gekennzeichnet, dass** das Koppelelement (30) oder die Verschlussklappe (20) im Bereich der zweiten Drehachse (34) einen Längsschlitz aufweist, über den die Verschlussklappe (20) gegenüber dem Koppelelement (30) lösbar und insbesondere aushebbar ist.

10. Gerätegehäuse gemäß einem der vorangegangenen Ansprüche, **dadurch gekennzeichnet, dass** zwei im Wesentlichen spiegelsymmetrische Koppelelemente (30) vorgesehen sind, die parallel zueinander ausgerichtet sind.

11. Gerätegehäuse gemäß einem der vorangegangenen Ansprüche, **dadurch gekennzeichnet, dass** das Koppelelement (30) bogenförmig ist, ein unterer Schenkel (30a) des Bogens in allen drei Positionen innerhalb des Gerätegehäuses verbleibt und ein oberer Schenkel (30b) des Bogens in den geöffneten Arbeitspositionen durch die Öffnung (14) aus dem Gehäuse geführt wird.

12. Gerätegehäuse gemäß einem der vorangegangenen Ansprüche, **dadurch gekennzeichnet, dass** der Zugangsbereich zu der Öffnung (14) durch eine Serviceperson in der dritten Arbeitsposition größer ist als in der zweiten Arbeitsposition.

13. Gerätegehäuse gemäß einem der vorangegangenen Ansprüche, **dadurch gekennzeichnet, dass** in der dritten Arbeitsposition der Öffnungswinkel (β) der Verschlussklappe (20) größer ist als der Öffnungswinkel (α) des Koppelelements (30).

## Claims

1. Equipment housing for a blood treatment device having a housing wall (10) and a closing flap (20) closing an opening (14) in the housing wall (10) in a first position and wherein a coupling element (30) is rotatably mounted with respect to the housing wall (10) via a first pivot (12) and the closing flap (20) is rotatably mounted with respect to the coupling element (30) via a second pivot (34), wherein the closing flap (20) can be disposed in at least one defined second working position in which the opening (14) in the housing wall is not closed,
wherein the closing flap (20) comprises at least parts (28,29) of an extra corporal blood treatment module comprising at least a pump, a flow meter, a valve, and/or a closing clamp,
**characterized in that**
the closing flap (20) is arranged such that it can be folded into the second working position due to the action of gravity.

2. Equipment housing according to claim 1 **characterized in that** the closing flap (20) can further be disposed in a defined third working position in which the opening (14) in the housing wall is not closed.

3. Equipment housing according to claim 1 or 2 **characterized in that** the housing wall (10) is substantially vertically aligned.

4. Equipment housing according to any of the preceding claims **characterized in that** the closing flap is not mounted with respect to the equipment housing via any further pivots or coupling elements.

5. Equipment housing according to any of the preceding claims **characterized in that** the closing flap (20) is not guided via linear guides with respect to the equipment housing.

6. Equipment housing according to any of the preceding claims **characterized in that** a locking mechanism (22, 32; 36) is provided between the coupling element (30) and the closing flap (20), and in particular the locking mechanism is arranged such that the closing flap (20) in the second working position is substantially vertically aligned when the locking mechanism (22, 32) is in an engaging position.

7. Equipment housing according to any of the claims 1 - 4 **characterized in that** a locking mechanism (22, 32; 36) is provided between the coupling element (30) and the closing flap (20), and in particular the locking mechanism is arranged such that the closing flap (20) in the second working position is aligned substantially parallel to the housing wall (10) when the locking mechanism (22, 32) is in a engaging position.

8. Equipment housing according to any of the preceding claims **characterized in that** the closing flap tends to pivot from the second working position into the third working position due to the action of gravity.

9. Equipment housing according to any of the preceding claims **characterized in that** the coupling element (30) or the closing flap (20) comprises a longitudinal slot in the region of the second pivot (34), via which the closing flap (20) can be released from the coupling element (30) and in particular removed.

10. Equipment housing according to any of the preceding claims **characterized in that** two substantially mirror-symmetrical coupling elements (30) are provided, aligned parallel to each other.

11. Equipment housing according to any of the preceding claims **characterized in that** the coupling element (30) is bow shaped and a lower limb (30a) of the bow remains within the equipment housing in all three positions, and an upper limb (30b) of the bow in the opened working positions is guided through the opening (14) out of the housing.

12. Equipment housing according to any of the preceding claims **characterized in that** the access area for a service engineer to the opening (14) is greater in the third working position than in the second working position

13. Equipment housing according to any of the preceding claims **characterized in that** in the third working position the opening angle (β) of the closing flap (20) is greater than the opening angle (α) of the coupling element (30).

## Revendications

1. Boîtier d'appareil pour un appareil de traitement de sang, comprenant une paroi de boîtier (10) et un clapet de fermeture (20), lequel ferme une ouverture (14) dans la paroi de boîtier (10) dans une première position, et un élément d'accouplement (30) étant monté de manière à pouvoir tourner par rapport à la paroi de boîtier (10) par le biais d'un premier axe de rotation (12), et le clapet de fermeture (20) étant monté de manière à pouvoir tourner par rapport à l'élément d'accouplement (30) par le biais d'un deuxième axe de rotation (34), le clapet de fermeture (20) pouvant être agencé dans une deuxième position de travail définie, dans laquelle l'ouverture (14) de la paroi de boîtier n'est pas fermée,
dans laquelle le clapet de fermeture (20) comprend au moins des parties (28, 29) d'un module de traitement de sang extracorporel comportant au moins une pompe et/ou un débitmètre et/ou une soupape et/ou une pince d'arrêt,
**caractérisé en ce que**
le clapet de fermeture (20) est agencé de telle sorte que il peut être pivoté dans la deuxième position de travail en raison de la force de gravité.

2. Boîtier d'appareil selon la revendication 1, **caractérisé en ce que** le clapet de fermeture (20) peut en outre être agencé dans une troisième position de travail définie, dans laquelle l'ouverture (14) de la paroi de boîtier n'est pas fermée.

3. Boîtier d'appareil selon la revendication 1 ou 2, **caractérisé en ce que** la paroi de boîtier (10) est orientée essentiellement verticalement.

4. Boîtier d'appareil selon l'une quelconque des revendications précédentes, **caractérisé en ce que** le clapet de fermeture n'est monté par rapport au boîtier d'appareil par le biais d'aucun axe de rotation et/ou d'aucun élément d'accouplement supplémentaire.

5. Boîtier d'appareil selon l'une quelconque des revendications précédentes, **caractérisé en ce que** le clapet de fermeture (20) n'est guidé contre le boîtier d'appareil par le biais d'aucun guide linéaire.

6. Boîtier d'appareil selon l'une quelconque des revendications précédentes, **caractérisé en ce qu'**un mécanisme d'encliquetage (22, 32; 36) est prévu entre l'élément d'accouplement (30) et le clapet de fermeture (20) et le mécanisme d'encliquetage est en particulier agencé de telle sorte que le clapet de fermeture (20) soit orienté essentiellement verticalement dans la deuxième position de travail lorsque le mécanisme d'encliquetage (22, 32) est dans une position d'encliquetage.

7. Boîtier d'appareil selon l'une quelconque des revendications 1 à 4, **caractérisé en ce qu'**un mécanisme d'encliquetage (22, 32; 36) est prévu entre l'élément d'accouplement (30) et le clapet de fermeture (20) et le mécanisme d'encliquetage est en particulier agencé de telle sorte que le clapet de fermeture (20) soit orienté essentiellement parallèlement à la paroi de boîtier (10) dans la deuxième position de travail lorsque le mécanisme d'encliquetage (22, 32) est dans une position d'encliquetage.

8. Boîtier d'appareil selon l'une quelconque des revendications précédentes, **caractérisé en ce qu'**en raison de la force de gravité, le clapet de fermeture a tendance à pivoter de la deuxième position de travail à la troisième position de travail.

9. Boîtier d'appareil selon l'une quelconque des revendications précédentes, **caractérisé en ce que** l'élément d'accouplement (30) ou le clapet de fermeture (20) présente, dans la région du deuxième axe de rotation (34), une fente longitudinale par le biais de laquelle le clapet de fermeture (20) peut être détaché et en particulier soulevé par rapport à l'élément d'accouplement (30).

10. Boîtier d'appareil selon l'une quelconque des revendications précédentes, **caractérisé en ce que** deux éléments d'accouplement (30) essentiellement en symétrie spéculaire sont prévus, lesquels sont orientés parallèlement l'un à l'autre.

11. Boîtier d'appareil selon l'une quelconque des revendications précédentes, **caractérisé en ce que** l'élément d'accouplement (30) est en forme d'arc et une branche inférieure (30a) de l'arc demeure à l'intérieur du boîtier d'appareil dans toutes les trois positions et une branche supérieure (30b) de l'arc est guidée hors du boîtier à travers l'ouverture (14) dans les positions de travail ouvertes.

12. Boîtier d'appareil selon l'une quelconque des revendications précédentes, **caractérisé en ce que** la région d'accès à l'ouverture (14) par une personne de service est plus grande dans la troisième position de travail que dans la deuxième position de travail.

13. Boîtier d'appareil selon l'une quelconque des revendications précédentes, **caractérisé en ce que**, dans la troisième position de travail, l'angle d'ouverture (β) du clapet de fermeture (20) est supérieur à l'angle d'ouverture (α) de l'élément d'accouplement (30).
